(19) **Europäisches Patentamt / European Patent Office / Office européen des brevets**

(11) **EP 4 681 600 A1**

(12) **EUROPEAN PATENT APPLICATION**
published in accordance with Art. 153(4) EPC

(43) Date of publication:
**21.01.2026 Bulletin 2026/04**

(21) Application number: **24795896.0**

(22) Date of filing: **16.04.2024**

(51) International Patent Classification (IPC):
**A61B 5/00** (2006.01)   **A61B 5/0531** (2021.01)
**A61B 5/0537** (2021.01)   **A61B 34/32** (2016.01)
**A61B 90/00** (2016.01)   **A61B 34/10** (2016.01)
**A61B 34/20** (2016.01)   **A61G 15/00** (2006.01)
**A61G 15/10** (2006.01)   **A61G 15/12** (2006.01)

(52) Cooperative Patent Classification (CPC):
**A61B 5/00; A61B 5/0531; A61B 5/0537;
A61B 34/10; A61B 34/20; A61B 34/32;
A61B 90/00; A61G 15/00; A61G 15/10;
A61G 15/12**

(86) International application number:
**PCT/CN2024/087865**

(87) International publication number:
**WO 2024/222515 (31.10.2024 Gazette 2024/44)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **28.04.2023   CN 202310483984
28.04.2023   CN 202321021790 U**

(71) Applicant: **Beijing Tashan Technology Co., Ltd.
Beijing 102308 (CN)**

(72) Inventors:
• **SUN, Tengchen
  Beijing 102308 (CN)**
• **ZENG, Fanyou
  Beijing 102308 (CN)**
• **WANG, Kai
  Beijing 102308 (CN)**

(74) Representative: **Yang, Shu
Withers & Rogers LLP
2 London Bridge
London SE1 9RA (GB)**

(54) **SKIN TESTING OPERATIONAL ROBOT SYSTEM HAVING EXTERNAL PRESSURE DETECTION FUNCTION**

(57)    The present invention relates to a skin detection and operation robot system with an external pressure detection function, which comprises a processing module, a detection end, an external actuator, an external pressure detection device, and an auxiliary positioning device for locating a specific part of the human body; the detection end is provided with a probe for the skin at a specific part of the human body, and

The sensor for detecting skin contact directly or indirectly abuts the front end of the skin; the external pressure detection device is configured to sense a first contact pressure between the front end and the skin through the external pressure detection device when the front end directly or indirectly abuts the skin; the external actuator is configured to drive the front end to move so as to adjust the first contact pressure ; the probe is configured with an internal pressure control device for controlling a second contact pressure between the sensor and the skin;

The processing module is coupled to the external actuator and the external pressure detection device respectively.

EP 4 681 600 A1

Figure 1

**Description**

**Technical Field**

Operation robot system with an external pressure detection function.

**Background Art**

Skin testing is of great significance to fields such as beauty and dermatology. With the development of technology, various types of skin testing probes have emerged, each with different uses. For example, some are used to detect the content of skin components (water/oil), some are used to detect skin elasticity, and some are used to detect skin gloss.

At present, a common problem of various skin detection probes is that the force, position and angle of each measurement are not uniform.EP88108905A The disclosed non-invasive acoustic test probe for skin elasticity, the operator presses the front end of the outer wall of the probe against the skin and then pushes the inner probes 4, 5, 6 Contact skin for detection, piezoelectric transducers 1, 2, 3 Sound pulses are transmitted to the probe, and the time span for the sound to travel between the probes is used to measure skin elasticity. In some practical scenarios where continuous tracking of skin parameters is required, the operator manually controls the contact of the probe with the skin, making it impossible to ensure that the same position, angle, and force are applied to the skin for each test. Consequently, it is difficult to distinguish whether differences in measured data are due to skin changes or changes in position, angle, or force.

US20020029924A1 proposes a measuring device for measuring the elastic properties of a surface structure. It is noted that for the value of the measurement result, it is crucial that the measurement results to be compared are taken from the same position /angle of the surface structure (skin) . Thus, a marking method is adopted, in which a circular flange on the outer wall is provided. 35 Set two holes on the circumference 40, for applying color markings on the surface structure, for example with a pen, so that measurements can be taken at greater time intervals at the same location with the same probe orientation, and furthermore, providing markings at predetermined angular distances from each other on the annular flange 36, with probe 2 Marking on the outside of the housing 38 Accordingly, the measuring device can be positioned reproducibly at the same measuring position and the same angular position on the surface structure.

US20020029924A1 It can solve the problem of the same position/angle, but it cannot solve the problem of the same pressure. Ensuring the same pressure is particularly important in skin measurement. The reason is that the skin itself has a certain elastic modulus andElasticity of theMasticatoryand Facial Expression Muscles in Patients with the Masticatory Muscle Pain》 Korean J Oral Med, Vol. 34, No. 3, 2009In a study, the elasticity of human skin was found to be approximately $0.70\pm0.46N$Whether it is a probe for measuring skin elasticity or a probe for other measurement purposes, during the test, in addition to the sensor in the probe (such as the probe mentioned above) needing to extend through the hole on the front face and press onto the skin, the front face of the probe shell (such as EP88108905A Protective case 12 Front face of US20020029924A1 annular flange 35 The front face of the sensor (the front face of the sensor) also presses against the skin during testing. As mentioned earlier, skin has an elastic modulus and is interconnected. The compression of the front face against the skin it covers will cause changes in the elasticity, moisture, and oil content of the skin within the front face's pores. The degree of change is related to the degree of compression, which in turn introduces errors and more direct interference to the sensor's detection. In actual testing, we found that even when measuring skin at the same location and angle within a short period of time, the measurement data obtained when the front face was not in contact with the skin (or just after contact) differed from the data obtained when the front face was pressed against the skin.

Therefore, it is crucial to ensure consistent external pressure (the pressure between the front face and the skin) during each skin test to maintain a stable measurement environment (consistent environmental standards) for the sensor in the inner ring. Furthermore, controlling internal pressure (the pressure between the sensor and the skin) is crucial for human safety , for example, preventing puncture injuries caused by excessive sensor pressure, thereby ensuring measurement safety.

On the other hand, skin testing equipment is primarily purchased by testing organizations and users, each with different product priorities and purchasing power. While ensuring measurement accuracy, users prioritize price, home use, and portability, while testing organizations favor full automation, convenience, and robust testing. This difference, reflected in manufacturing, directly impacts product design requirements and manufacturing costs.

**Summary of the Invention**

The purpose of the present invention is to provide a fully automatic control robot system suitable for a detection agency, which can fully automatically and conveniently achieve uniform force, position and angle for each skin detection. Maintain a stable measurement environment for the sensor during each measurement to avoid measurement errors caused by different pressures, especially external pressures, while ensuring measurement safety.

To this end, a skin detection and operation robot system with an external pressure detection function is provided, comprising a processing module, a detection end, an external actuator, an external pressure detection device, and an auxiliary positioning device for locating a specific part of the human body; the detection end is provided with a probe for detecting the skin at the specific part of the human body, and a front end surface of the probe directly or indirectly against the skin when a sensor for detecting the skin contacts the skin; the external pressure detection device is configured to sense a first contact pressure between the front end surface and the skin through the external pressure detection device when the front end surface directly or indirectly against the skin; the external actuator is used to drive the front end surface to move so as to adjust the first contact pressure; the probe is provided with an internal pressure control device for controlling a second contact pressure between the sensor and the skin; and the processing module is coupled to the external actuator and the external pressure detection device, respectively.

The present invention has the following advantages:

(1) After locating a specific part of the human body through the auxiliary positioning device, the external actuator is controlled to drive the detection end at the same position/ angle for detection. During the process, the external pressure detection device is used to feedback the first contact pressure (external pressure ) between the front end surface and the skin. The first contact pressure is adjusted to be consistent with the previous measurement through the external actuator, maintaining the sensor in a uniform external pressure measurement environment in each measurement to avoid introducing measurement errors;

(2) between the sensor and the skin to ensure measurement safety;

(3) The overall operation process is fully automated, convenient and stable,
and is suitable for testing institutions.

In the present invention, the external actuator is configured as a movable mechanical arm, which drives the detection end as a whole to perform ZXY Movement with variable direction and angle can achieve high-freedom movement control; the sensor can be configured to be hidden under the front end surface, extended to touch the skin when in use, and hidden inside for protection when not in use, or the sensor can be fixed on the front end surface and the outer wall can be flush with the front end surface.

As an improved solution, the probe is equipped with an internal pressure detection device coupled to the processing module. The internal pressure detection device is used to detect the second contact pressure (internal pressure) between the sensor and the skin, achieving the purpose of dual internal and external detection. Furthermore, the internal pressure control device is configured as an internal actuator coupled to the processor. The internal actuator is provided in the probe and can be a component such as a motor or a micro motion device to drive the sensor on the probe.

The sensor moves precisely to adjust the second contact pressure between the sensor and the skin. By configuring an internal actuator with the detection feedback of the second contact pressure, precise control of the internal pressure is achieved under the control of the unified external pressure, ensuring that the internal pressure used in each measurement is also consistent. At this time, the external actuator realizes large-scale, high-degree-of-freedom and angle movement control, and the internal actuator is used for fine-tuning. In the present invention, the internal pressure control device can also be used in conjunction with a prompt device, such as when it is detected that the internal pressure is consistent with the last time, it prompts the operator to stop adjusting the sensor position. In another embodiment, the internal pressure control device can also be configured as an elastic body such as a spring. The sensor is fixed to the probe through the elastic body to elastically control the second contact pressure within a set range. Compared with the above-mentioned solution that can achieve precise control, this solution achieves general control of the internal pressure ( elastic control within a certain range) and brings structural and cost advantages.

As another improvement scheme, the auxiliary positioning device is configured as a fixed bracket to assist in fixing a specific part of the human body, such as a bracket for supporting the chin on an instrument for detecting eye vision. During the detection process, the bracket is used to confirm the position of the human body, and the probe is accurately moved to the detection position for measurement through an external actuator. More preferably, the fixed bracket is further configured as a movable folding chair, which moves along the track and sets a double limit position to realize different sitting and lying detection methods. At the same time, a neck fixing device is added to the movable folding chair corresponding to the human neck to realize positioning. And/or the auxiliary positioning device is configured as a visual scanning imaging system coupled to the processing module, and the visual scanning imaging system is connected to the processing module through the visual scanning imaging system. 3D Scanning imaging technology scans specific areas of the human body to determine the target detection location. The processing module controls the movement of the robotic arm based on the target detection location, thereby precisely moving the probe to the detection location for measurement. Furthermore, the visual scanning imaging system is composed of three sets of scanning cameras, which achieve all-round detection of the three sides of the human body, thereby accurately constructing a human body model. The three sets of scanning cameras are installed on a main frame with adjustable height to match people of different body shapes for precise scanning and positioning.

In the present invention, the number of external pressure detection devices is configured to be at least two, and they are arranged around the probe to take into account various circumferential positions and ensure detection uniformity.

Furthermore, in order to avoid the measurement error of the sensor caused by adding an object between the sensor and the skin, the internal pressure detection device uses a capacitive pressure sensing component to indirectly measure the contact pressure (it is not suitable to use a resistive type to avoid the need to place a pad between the electrode and the skin).

Pressure can be indirectly reflected by area and/or distance, for example:

The solution of using distance to reflect pressure can be implemented as follows: the pressure sensing component is configured to include at least a first distance detection electrode and a second distance detection electrode. One side of the first substrate is used to accommodate the sensor. The first distance detection electrode is fixed to the side of the first substrate away from the sensor. The second distance detection electrode is arranged along the movement direction of the first substrate and is at least partially or fully aligned with the first distance detection electrode. A capacitance-to-digital conversion circuit (CDC) couples the first and second distance detection electrodes to obtain mutual capacitance between them. A processing module is configured to output information about the movement distance of the first substrate based on the mutual capacitance between the first and second distance detection electrodes. Because the sensor is in close contact with the skin, the movement of the first substrate is proportional to the second contact pressure. Utilizing this characteristic, during operation, the movement of the first substrate changes the distance between the first and second distance detection electrodes, thereby causing a change in mutual capacitance between them. The processing module calculates the movement distance of the first substrate based on the change in mutual capacitance and converts it into pressure data, achieving indirect measurement. In this case, the distance detection electrode for measuring pressure is located on one side of the first substrate, and the sensor is located on the other side, so that the two do not interfere with each other. More preferably, in order to avoid the first distance detection electrode from being misaligned or tilted relative to the second distance detection electrode, the pressure sensing component is provided with a guide column, and the first substrate is guided by the guide column. The specific structure can be configured as the first substrate is sleeved on the guide column.

For solutions that use area or even area and distance to reflect pressure, patent CN202223551426.5 can be used.The two-dimensional force structure shown here features a cylindrical or semi-cylindrical curved elastic upper electrode within a strip-shaped flexible multifunctional layer, fixed to the side of the first substrate away from the sensor (with the cylindrical or semi-cylindrical curved surface facing away from the sensor). Below the upper electrode, at least two lower electrodes, distributed on either side of the strip, are positioned. Different capacitances are formed between the upper and lower electrodes to reflect force components in different directions. An insulating layer is provided between the upper and lower electrodes, and the downward projection of the upper electrode covers at least a portion of the area of each lower electrode. When the first substrate moves, the upper electrode deforms in the radial direction of the strip, causing the upper electrode to change its contact area with the insulating layer, thereby reflecting pressure changes.

Alternatively, using patents CN201910370967.1 The three-dimensional force structure shown achieves higher resolution measurement. In this solution, indirect measurement is also achieved and the pressure detection and the skin detection of the sensor do not interfere with each other.

Another problem with skin detection probes is that there are many types of probes, and the interfaces of each probe are not unified, which leads to the problem of non-universality of the back-end docking equipment. In this regard, as another improvement solution, the detection end is provided with a standard connector for interchangeably docking with different probes for detecting human skin, thereby realizing the corresponding detection function of the probe and solving the problem of interface unification.

More specifically, the skin detection probe is used to detect skin components (water/oil). The water content in the skin can form moisture on the skin surface, while the oil helps the skin to lock in moisture and inhibit bacteria. The traditional method of detecting water or oil in the skin mostly uses thin film measurement method, which uses the thin film to absorb water or oil and then conduct standard comparison through optical means, such as the U.S. patent4,532,937 Disclosed microporous membrane adhered to the skin for absorbing sebum; U.S. Patent No. 5,119,828 The disclosure discloses the use of a microporous hydrophobic polymer membrane, which is opaque when the pores are filled with gaseous material and becomes translucent when the pores are filled with sebum, and uses this property to perform optical measurements; Alternatively, German Patent DE29700324U1 discloses skin analysis and evaluation through test films, etc. The thin film measurement method is an indirect measurement method that requires water or oil to be transferred to the test film before testing. The transfer process is prone to errors due to interference from various uncertain factors. For this reason, solutions using direct measurement methods to test skin moisture have appeared on the market, such as CK The principle of the corneal measurement instrument is to use a capacitive measurement method. The mutual capacitance electric field formed by two measuring electrodes penetrates the human skin. The addition of water causes the dielectric constant in the sensing area to change, and the mutual capacitance value measured reflects the moisture content in the skin. The main problem with this method is that we want to test the series capacitance between the measuring electrode and the skin. The skin is the outer layer of the human body, and beneath the skin there are various conductive substances such as blood. The collection of all these substances will form the distributed capacitance (self-capacitance) $C_w$ formed by the human body to the ground. Whether measuring self-capacitance or mutual capacitance, the self- capacitance of the human body will be included .$C_w$, resulting in series capacitance $C_a$ Inaccurate measurement affects the accuracy of measurement, and invasive methods such as cutting part of the skin are used to isolate the human body's self-capacitance. $C_w$, due to the loss of skin activity after cutting , it will also cause measurement errors. Therefore, for the probe for detecting skin components (water/oil) in the skin detection probe, that is, the skin component detection module, its sensor can be configured to include at least a first measuring electrode and a second measuring electrode. The first measuring electrode and the second measuring electrode are used to contact the skin through the insulating layer, and the series capacitance $C_{a1}$ between the first measuring electrode and the human body. and the series capacitance between the second measuring electrode and the human body $C_{a2}$ The ratio is configured to set a known proportional coefficient k . The skin detection operation robot system is provided with a capacitance- to-digital conversion circuit. The capacitance-to-digital conversion circuit couples each measuring electrode and obtains a first capacitance and a second capacitance. The first capacitance is configured to be one of a first self-capacitance measurement value obtained by the first measuring electrode, a second self-capacitance measurement value obtained by the second measuring electrode, a third self-capacitance measurement value obtained by connecting the first measuring electrode and the second measuring electrode in parallel, and a first mutual capacitance measurement value obtained by the first measuring electrode and the second measuring electrode. The second capacitance is configured to be one of the other three. A processing module is used to construct a distributed capacitance of the human body to the ground based on the first capacitance . $C_w$, the corresponding series capacitance as variables, and the first equation based on the second capacitance to construct the human body to ground distribution $C_w$, the second equation with the corresponding series capacitance as the variable, using the equation system composed of the first and second equations and the proportional coefficient k Calculate the series capacitance $C_{a1}$ or series capacitance $C_{a2}$ To output the component information in the skin. In actual scenarios, when the skin component detection module is detecting, the first measuring electrode and the second measuring electrode are closely attached to the skin through the insulating layer, and the distance between the electrodes and the skin is fixed. In this way, only the area ratio of the first measuring electrode and the second measuring electrode after manufacturing is set , and the proportional coefficient can be set by using the structure setting. K D etermine (the series capacitance of one is $C_a$, The other series capacitor is $k*C_a$ ), on this basis, by measuring the self-capacitance or mutual capacitance of the electrode twice , since the self-capacitance or mutual capacitance is composed of $C_w$ and $C_a$ , a function equation of $C_w$ and $C_a$ can be constructed for each measurement , and the equation system composed of the two equations can be used to calculate the Cw Perform elimination to construct Ca A monotonic function of the first and second capacitances is then used to solve the first and second capacitances obtained from the two measurements. Ca . Due to Cw is eliminated, so it can be eliminated Cw The measurement error caused by this is then accurately measured and calculated. Ca To reflect the content of skin components. And, using capacitance digital conversion circuit (CDC , ) such as ADI7142 and ADI7147 use Δ-Σ modulation to directly convert the measured capacitance value into a digital value by charging and discharging the measured capacitance multiple times and comparing it with the reference capacitance (see: US Patent Number: 5,134,401).

The sensitivity of capacitance measurement can be increased to 1fflevel, which easily meets the measurement system's requirements for capacitance measurement sensitivity. In particular, the design of these chips has multiple channels, which makes circuit design simple and convenient, thereby effectively reducing costs and installation difficulty.

As a further improvement to this improved solution, the proportional coefficient k is configured to be equal to1, or not equal to1. k The solution equal to 1 can be achieved by setting the first measuring electrode and the second measuring electrode to correspond to the normal direction of the human body.

The same projection area and the same distance between the first measuring electrode and the human body as the second measuring electrode are achieved. This solution is more conducive to reducing computing power and improving detection speed, and is a preferred solution. The solution of k not being equal to 1 can be achieved by setting different areas and/or spacings, for example, setting the area of the first measuring electrode to half the area of the second measuring electrode, or setting the spacing between the first measuring electrode and the human body to half the spacing between the second measuring electrode and the human body. 1/3wait.

As a further improvement to this improved solution, the first capacitance is configured as a first self-capacitance measurement value or a second self-capacitance measurement value, and the second capacitance is configured as a first mutual capacitance measurement value. In this case, if one of the first self-capacitance measurement value and the second self-capacitance measurement value is $c_S$ and its corresponding series capacitance is $c_a$ , then the value of the other series capacitance is $k * c_a$ ; the calculation method of $c_a$ is further configured as:

$$C_a = k_1 * \frac{k*C_s + k*C_x + \sqrt{C_s^2*k^2 - 2C_s*C_x*k^2 + C_x^2*k^2 + 4*C_s*C_x*k}}{2*k} \pm k_2;$$

Where $c_x$ is the first mutual capacitance measurement value, $k_1$ is 0.1-0.9, $k_2$ is the allowable error value , set to $\pm5\%$ of the measured value. In this solution, the first capacitor is configured as self-capacitance, and the second capacitor is configured as mutual capacitance. A more accurate measurement result is obtained by converting self-capacitance into mutual capacitance.

Alternatively, as an alternative to this improved solution, the first capacitance is configured as the first self-capacitance measurement value or the second self-capacitance measurement value, and the second capacitance is configured as the third self-capacitance measurement value. In this case, one of the first self-capacitance measurement value and the second self-capacitance measurement value is $C_{S1}$, and its corresponding series capacitance is $C_a$ . Then, the value of the other series capacitance is $k * C_a$ , and the calculation method of $C_a$ is further configured as:

$$C_a = k_1 * \frac{C_{S1} * C_{S2} * k}{k * C_{S2} - k * C_{S1} + C_{S2} - C_{S1}} \pm k_2;$$

Where $C_{S2}$ is the third self-capacitance measurement value, $k_1$ is between 0.1 and 0.9, and $k_2$ is the tolerance, set to $\pm5\%$ of the measured value. In this solution, the first capacitor is configured as a self-capacitance, and the second capacitor is configured as a self-capacitance with a variable area (by combining two electrodes in parallel using an analog switch). This dual self-capacitance and variable area measurement method yields more accurate results.

As a further improvement to this improved solution, the spacing between the first measuring electrode and the second measuring electrode is configured to be 0.1mm-2mm, thereby ensuring that the two electrodes sense temperature and humidity changes in the same space, avoiding interference caused by large temperature and humidity differences at the two electrodes due to excessive distance.

In the present invention, the components can be water or oil, and the water and oil in the skin are at different depths.

The water-oil-deep characteristic is achieved by detecting different depths. At this time, when measuring oil and fat , the water and human body self-capacitance can be used together as the sensor by controlling the penetration depth of the electric field line. $C_w$ , or when measuring the water content of shallow layers (such as epidermal tissue), the water content of deeper layers (such as dermal tissue and/or subcutaneous tissue) and the body's self-capacitance can be used together as the water content of shallow layers (such as epidermal tissue) by controlling the penetration depth of the electric field lines. $C_w$. And/or , water-oil differentiation can be achieved by changing the excitation frequency (using an excitation frequency that is sensitive to water, an excitation frequency that is sensitive to oil).

Based on this, as a further improvement to this improved solution, at least three measurement electrodes (including first and second measurement electrodes ) can be configured at different locations. The capacitance-to-digital conversion circuit couples the measurement electrodes via an analog switch array to selectively combine any at least two measurement electrodes to form a pair of electrodes for detecting mutual capacitance. On the one hand, the measurement electrodes are composed of multiple groups of mutual capacitances. Switching through the analog switch array enables detection of components at different locations, thereby averaging and reducing errors caused by positional differences between measurements. On the other hand, based on this structure, at least two pairs of electrode groups can be combined, where the electric field lines of the mutual capacitance formed by each pair of electrode groups have different depths, thereby varying the penetration depth of the electric field lines. Specifically, this structure can adopt a variable area and/or variable spacing approach, whereby the area or spacing of the two electrodes in each pair of electrode groups is varied. This results in a difference in the depth of the electric field lines of the mutual capacitance formed by each pair of electrode groups, thereby enabling detection at different depths or in different layers of skin tissue. Among them, the variable area scheme, for example, setting six electrodes 1-1, 1-2, 1-3, 1-4, 1-5, 1-6, CDC Measure1-2, 1-3 via analog switch array The mutual capacitance between them, at this time the mutual capacitance electric field line penetration depth is shallow, and then by 1-1, 1-2 Combined (parallel ), 1-3, 1-4 Merge and measure the mutual capacitance between the two merged electrodes. The area of the merged electrodes increases and the penetration depth of the electric field lines becomes deeper. At this time, the skin tissue at different depths in the same position can be tested . Alternatively, first detect 1-1, 1-2 The mutual capacitance between 1-1, 1-2merge, 1-3, 1-4Combine the mutual capacitance after measuring the variable area to test the skin tissue at different depths at different positions. For the variable spacing scheme, the six electrodes can be equally spaced or unequally spaced. For example, in the equal spacing setting, 1-1, 1-2 Test epidermal tissue, 1-1, 1-4 Test dermis tissue, 1-1, 1-6 Test subcutaneous tissue; or use 1-1, 1-2 in different spacing to simplify wiring Test epidermal tissue, 1-3, 1-4 test

Dermis, 1-5, 1-6Test subcutaneous tissue. As can be seen from the above, based on the configuration of at least three measuring electrodes distributed in different positions, CDC In conjunction with the analog switch array, multiple functional purposes can be achieved : (1) conveniently reducing the error caused by the position difference between the measurement times by averaging ; (2) conveniently realizing variable spacing and/or variable area through combination to detect different depths, including different depths at the same position and/or different depths at different positions. In the present invention, the analog switch array can use an analog signal router to achieve simple and convenient free combination switching. , The information of analog signal router can be referred to patent CN202110957486.8, which will not be repeated here.CDC integration, such as usingCN202110956246.6 Ruby chip, to achieve twenty four High-speed CDC, effective resolution21.9bit reaches 0.5ms conversion time and high-precision tactile signal acquisition and encoding.

More preferably, the electrode group is configured to include at least two of the following: a first electrode group, wherein the spacing and/or area of the two electrodes in the first electrode group is configured so that the depth of the electric field lines of the mutual capacitance can penetrate into the epidermal tissue of the skin; a second electrode group, wherein the spacing and/or area of the two electrodes in the second electrode group is configured so that the depth of the electric field lines of the mutual capacitance can penetrate into the dermal tissue of the skin; and a third electrode group, wherein the spacing and/or area of the two electrodes in the third electrode group is configured so that the depth of the electric field lines of the mutual capacitance can penetrate into the subcutaneous tissue of the skin . The epidermal tissue, dermal tissue, and subcutaneous tissue together constitute the skin, and by configuring the electric field lines to penetrate into at least two or even three of them, the skin can be effectively protected. CDC The averaging with the analog switch array can achieve the purpose of reflecting the skin component parameters more comprehensively and closely, and can selectively detect the tissue status of different layers. In the above-mentioned

scheme of detecting different depths or detecting different layers of skin tissue by means of variable area and/or variable spacing, the basic principle is to follow CDC The premise is that the frequency of the excitation signal output to the measuring electrode is configured as a fixed frequency. In another solution for changing the detection depth, the frequency of the excitation signal can also be configured in at least two ways, for example, between two measuring electrodes with a fixed spacing and a fixed area, such as the first measuring electrode and the second measuring electrode, by configuring the software in the ruby chip so thatA The excitation signal of the time period operates at the first frequency. B The time period operates at the second frequency. Due to the different frequencies, the penetration depth of the mutual capacitance electric field lines between the first measuring electrode and the second measuring electrode will also change under the action of the skin effect, thereby achieving measurements at different depths. It is worth noting that, of course, it is also possible to further use a frequency-varying method based on the variable area and/or variable spacing .

This approach allows for finer-grained depth control. Furthermore, it's more preferable to configure the selected frequencies to be sensitive to different components in the skin. For example, the first frequency could be sensitive to water, while the second could be sensitive to oil, thereby achieving water-oil differentiation.

As a further improvement to this improved solution, the skin component detection module also includes a standard liquid storage device; the standard liquid storage device at least includes a sealed cavity, a standard liquid corresponding to the component disposed in the sealed cavity for calibration or differential measurement, and a liquid detection electrode for detecting the capacitance value of the standard liquid under different environments; a capacitance-to-digital conversion circuit coupled to the liquid detection electrode; and a processing module for correcting the capacitance (self-capacitance, mutual capacitance) obtained by the capacitance-to-digital conversion circuit based on the capacitance value of the standard liquid . When the measured component is water, the standard liquid is standard water; when measuring grease, the standard liquid is converted to grease. The liquid detection electrodes are configured to include at least two and are distributed on the outer wall of the sealed cavity. The capacitance value of the standard liquid in the cavity is detected by the mutual capacitance of the two liquid detection electrodes .

In this improved solution, the method for using the standard liquid for calibration further includes detecting the difference between the capacitance value of the standard liquid at the current moment and the capacitance value at the initial moment, and using the change in the reference reflected by the difference to correct the capacitance obtained by the CDC. For example, assuming that the volume of the standard liquid is configured to correspond to the full scale, the capacitance value of the standard liquid detected initially is A, means the capacitance value A Corresponding to the full scale, if the capacitance value of the standard liquid detected at the current moment is not A And for B means that the environment (such as temperature and humidity) changes and causes the capacitance to change. At this time, the difference between the capacitance value of the same volume of standard liquid from A to B means that the reference has changed.Bcorresponds to the full scale, so it is necessary to CDC The obtained capacitance is corrected corresponding to the reference variation.

In this improved solution, the method of using standard liquid for differential measurement further includes differentially comparing the capacitance value measured by the standard liquid detection electrode with the capacitance value measured by the user through a digital circuit CDC, which can reduce the measurement error (common-mode interference ) caused by changes in environmental factors. The standard liquid is stored in a sealed cavity and does not need to be replaced frequently, reducing the user's

calibration operation when performing skin testing , achieving calibration while measuring, and making the operation easier.

**EP 4 681 600 A1**

**BRIEF DESCRIPTION OF THE DRAWINGS**

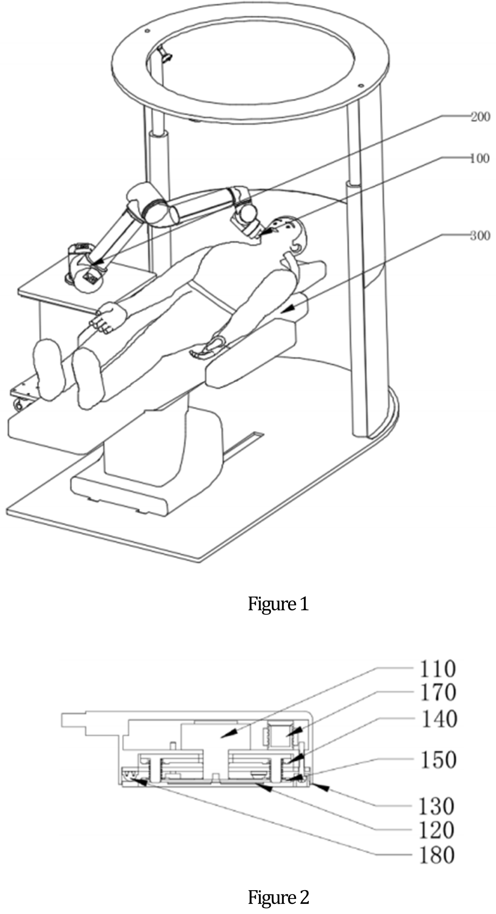

Figure 1 Shows the overall structural diagram of the skin detection operation robot system;

Figure 2 shows a schematic diagram of the detection end structure;

Figure 3-1 shows the first perspective of the external pressure detection device;

Figure 3-2 shows a second viewing angle of the external pressure detection device;

Figure 4 Shows a schematic structural diagram of the internal pressure detection device;

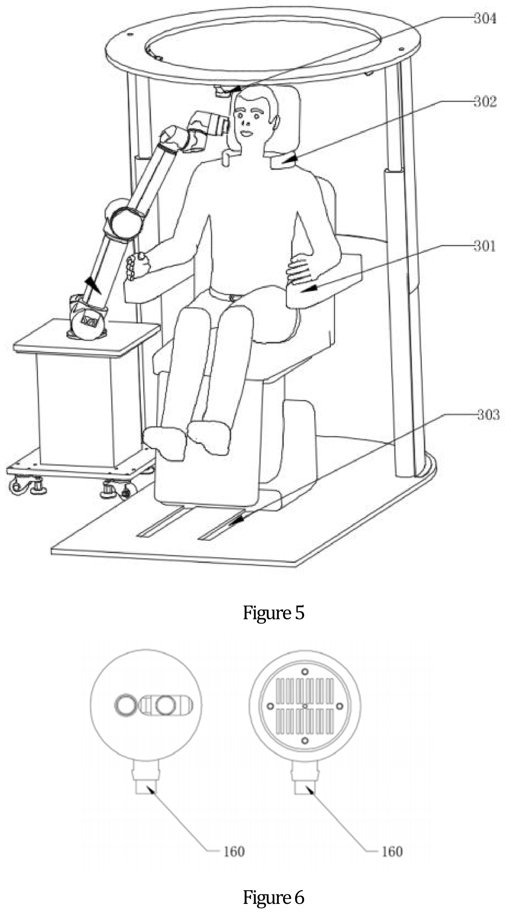

Figure 5 Shows a schematic structural diagram of the auxiliary positioning device;

Figure 6 Shows a schematic diagram of the standard interface structure of different detection ends;

Figure 7-1 shows a schematic diagram of the local structure of the detection sensor;

Figure 7-2 The equivalent schematic diagram of the distributed capacitance between the detection electrode and the human body to the ground is shown;

FIG7-3 shows the self-capacitance measurement value of the first capacitance configuration as the first measurement electrode $C_{s1}$ and the second capacitance configuration as the second measurement electrode Capacitance distribution diagram when measuring the self-capacitance value of $C_{s2}$ ;

FIG7-4 shows a capacitance distribution diagram when the first capacitance is configured as the self-capacitance measurement value of the first measuring electrode Cs1 and the second capacitance is configured as the mutual capacitance between the first detection electrode and the second detection electrode;

FIG7-5 shows the self-capacitance measurement value of the first capacitance configuration as the first measurement electrode $C_{s1}$, the second capacitance configuration as the first measurement electrode $C_{s1}$ With the second measuring electrode $C_{s2}$ Capacitance distribution diagram of self-capacitance after parallel connection;

Figure 8-1 shows a schematic diagram of the structure of electrode groups with different spacing or areas for testing depth;

Figure 8-2 The depth structure intention of the test is shown with the excitation signals of different frequencies;

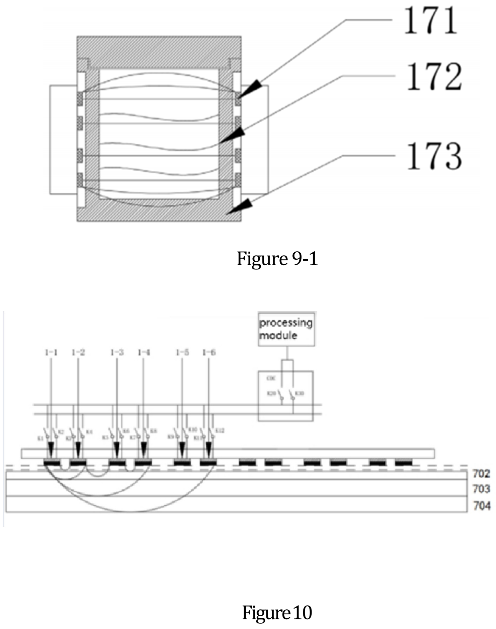

Figure 9-1 shows a schematic diagram of the structure of a standard liquid storage device;

Figure 10 A schematic diagram of a switch array for testing skin components at different depths using a combination of multiple electrodes is shown.

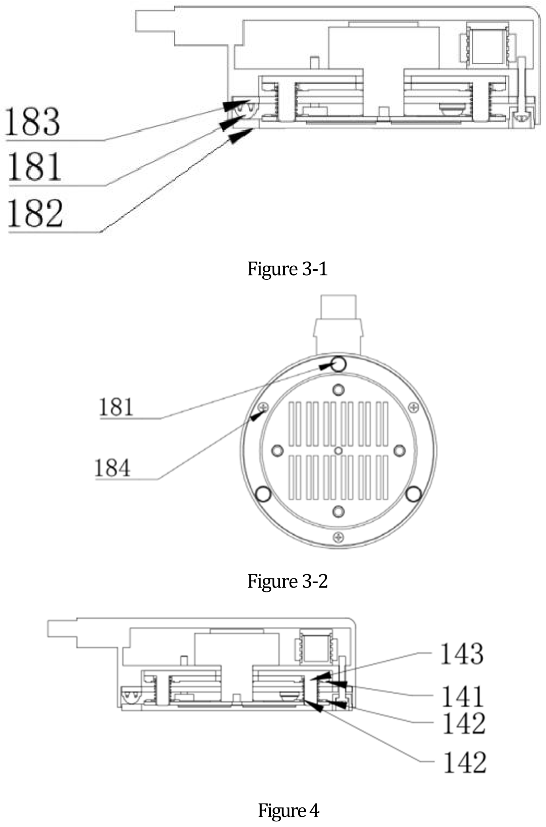

The technical solutions in the embodiments of the present invention will be described clearly and completely below with reference to the accompanying drawings in the embodiments of the present invention.
As shown 1As shown in the figure, the skin detection operation robot system with external pressure detection function mainly includes the detection end 100. External actuator200. Auxiliary positioning device300. External actuator as an example200Can A robotic arm is used.
As shown 2 As shown, the detection end 100 Including internal brake 110. Detection sensor 120. Auxiliary positioning shell 130. Internal pressure detection device 140. Front-end processing module 150. External pressure detection device 180. Detection end 100 by auxiliary positioning device 300 Initial positioning, through external actuator 200 The external pressure detection device 180 is adjusted so that the outer surface thereof contacts or presses against the human skin. When the preset first contact pressure range is reached, detection is started.
As shown 3-1, Figure 3-2 ,the external pressure detection device shown is composed of an external pressure sensor 181, the external pressure first base 182, the external pressure second base 183, the external pressure positioning screw 184, the external pressure sensor 181 needs at least three to be evenly distributed around the detection center, the external pressure positioning screw 184 There are at least three pressure sensors evenly distributed around the detection center and connected to the external pressure sensor. 181 When the user's skin contacts and resists the external pressure, the first substrate 182 The outer surface (front end face of the detection end ) of the external pressure first substrate 182 Relying on set screws 184Move parallel to press the external pressure sensor 181When the preset external pressure value is reached, the external actuator 200 Stop the adjustment action. In this embodiment, the external pressure sensor is pressed 181 Can be used as patent CN202223551426.5 The two-dimensional force structure shown, or the patented The three-dimensional force structure shown in CN201910370967.1 is realized.

As shown4As shown, the internal pressure detection device 140Contains a first distance detection electrode 141. Second distance detection electrode 142. Pressure detection guide column 143. Pressure detection elastomer 144, detection sensor 120 By evenly distributed actuators 110 surrounding 2-4 Pressure detection guide column 143Implementing a pressure sensor 120Y direction movement, and limit tilt and rotation, detection sensor 120 By means of the inner actuator 110 Axial micro-movement is possible by pressure detection of elastic body 144 Provides axial thrust to fix the detection sensor 120 With internal actuator 110 The relative position of the installation plane and the detection electrode according to the first distance 141 and the second distance detection electrode 142 The change in the mutual capacitance value formed between the sensors is detected 120Compared with the distance between the front end of the detection end and the pressure between the skin , the number of steps of the inner actuator 110 is adjusted according to the feedback information of the pressure value obtained,

and the detection end is accurately controlled. 100The contact pressure with the user's skin reaches a preset value.

As shown 5 As shown, the auxiliary positioning device 300 Mainly includes mobile seats301. Sliding Track302. Neck Fixation Device 303, 3D Scanning Imager 304, the user relies on the mobile seat 301 With limited power

Energy sliding track302To achieve the position positioning of sitting or lying down, the user can realize the initial positioning of the head through the neck fixing device 303. At least one 3Group3DScanning Imager304, realize multi-angle and multidirectional scanning of the user, and combine the three sets of data to make an accurate human body model of the user 1Build and confirm the relative coordinates of the test points. 3D Scanning Imager 304 Scan the user and build an accurate human body model 2. Through the human body model1With human body model2 Compare the test points to find the exact test points and send them to the actuator through the processing module. 200 Send instructions to move or rotate to find the previous test position, achieve dynamic and accurate repeated positioning, and thus reduce measurement errors caused by repeated positioning deviations

As shown6As shown, a variety of detection probes can be set for different skin detection items. Different detection probes and robotic arms 201 The connection and fixation can use the standard interface 160, which contains signal output and mechanical fixing buckle, so that the detection end of different skin detection items can be realized. 100 interchangeability.

As shown 7-1As shown, the detection sensor 120 of the detection end 100Contains at least two detection electrodes 121, each detection electrode has an insulating layer 122, the insulating layer 122 Including but not limited to attaching insulating tape or electrode surface coating, the detection electrode 121 at least includes a first measuring electrode $C_{s1}$, second measuring electrode $C_{s2}$, $C_{a1}$The first measuring electrode $C_{s1}$Series capacitance with the human body, $C_{a2}$The second measuring electrode $C_2$The series capacitance between the first measuring electrode and the human body$C_{s1}$ With the second measuring electrode$C_{s2}$Configured for known scale factors K, when the proportional coefficient is configured as 1 When the first measuring electrode and the second measuring electrode have the same area and the same distance from the human skin. The skin detection operation robot system is provided with a capacitance- to-digital conversion circuit (CDC), which couples each measuring electrode and obtains a

first capacitance and a second capacitance. The first capacitance is configured as one of a first self- capacitance measurement value obtained by the first measuring electrode, a second self-capacitance measurement value obtained by the second measuring electrode, a third self-capacitance measurement value obtained by connecting the first measuring electrode and the second measuring electrode in parallel, and a first mutual capacitance measurement value obtained by the first measuring electrode and the second measuring electrode, and the second capacitance is configured as one of the remaining three.

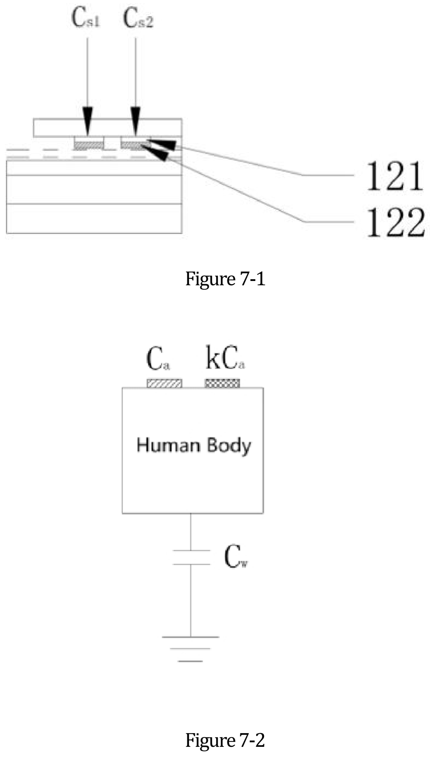

Figure 7-2 shows the equivalent diagram of the detection electrode and the distributed capacitance of the human body to the ground. For the convenience of conversion and understanding in the following text, for example, the first measurement electrode$C_{s1}$The series capacitance between the body and the$C_a$, then the second measuring electrode $C_{s2}$ Series capacitance with the human body$C_{a2}$ It can be expressed ask $C_a$, the human body can be equivalent to a capacitor to the ground $C_w$.

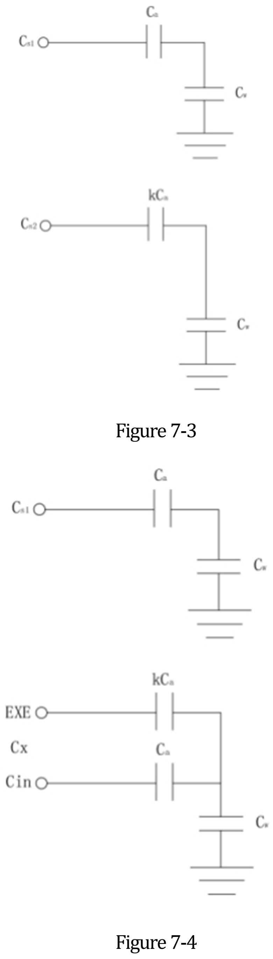

Figure 7-3 The two self-capacitances are used to measure $C_a$ In the method shown in FIG7-3, the first measuring electrode $C_{s1}$ The obtained self-capacitance measurement value is used as the first capacitance, and the processing module constructs the distributed capacitance of the human body to the ground based on the first capacitance. The first equation with $C_w$ and the corresponding series capacitance as variables can be used to obtain the distributed capacitance

$C_w$ of the human body to the ground $= \dfrac{C_{s1}*C_a}{C_a-C_{s1}}$ ; To obtain two measuring electrodes $C_{s2}$ The self-capacitance measurement value is taken as the second capacitance, and a human body-to-ground distribution is constructed based on the second capacitance. $C_w$, the second equation with the corresponding series capacitance as the variable , we can get the equation $\dfrac{1}{C_{s2}} = \dfrac{1}{k*C_a} + \dfrac{1}{C_w}$ , The equation system composed of the first equation and the second equation and the proportional coefficient k, and then the elimination can be performed to obtain $C_a$ , $= \dfrac{C_{s1}*C_{s2}*k-C_{s1}*C_{s2}}{k*C_{s2}-k*C_{s1}}$ , and then the component information in the skin is output.

Figure 7-4 Shows the use of self-capacitance and mutual capacitance to measure $C_a$ In the method shown in FIG7-4 , for example, the first measuring electrode $C_{s1}$ The obtained self-capacitance measurement value Cs $_{is}$ used as the first capacitance, and the equation can be obtained: $\dfrac{1}{C_s} = \dfrac{1}{C_a} + \dfrac{1}{C_w}$ , Derived $C_w = \dfrac{C_s*C_a}{C_a-C_s}$ , The second capacitor is configured as a first measuring electrode $C_{s1}$ With the second measuring electrode $C_{s2}$ The first mutual capacitance measurement $C_x$ between the excitation EXEis the amplitudeVe square wave, due tokC and $C_w$ The partial pressure ofCatoCin The excitation voltage drops toVe*k*$C_a$/ (k*Ca+Cw ) , the essence of capacitor charging and discharging is charge transfer. According to Q= CU , the amount of charge is proportional to the voltage, so the actual measured mutual capacitance value is expressed as $C_x = \dfrac{k*C_a*C_a}{k*C_a+C_w}$ , $C_w$ Substituting into the mutual capacitance equation, we can get the $C_a$ The calculation method is configured as $C_{a1} = \dfrac{k*C_s+k*C_x+\sqrt{C_s^2*k^2-2C_s*C_x*k^2+C_x^2*k^2+4*C_s*C_x*k}}{2*k}$ , and further outputs the component information within the skin.

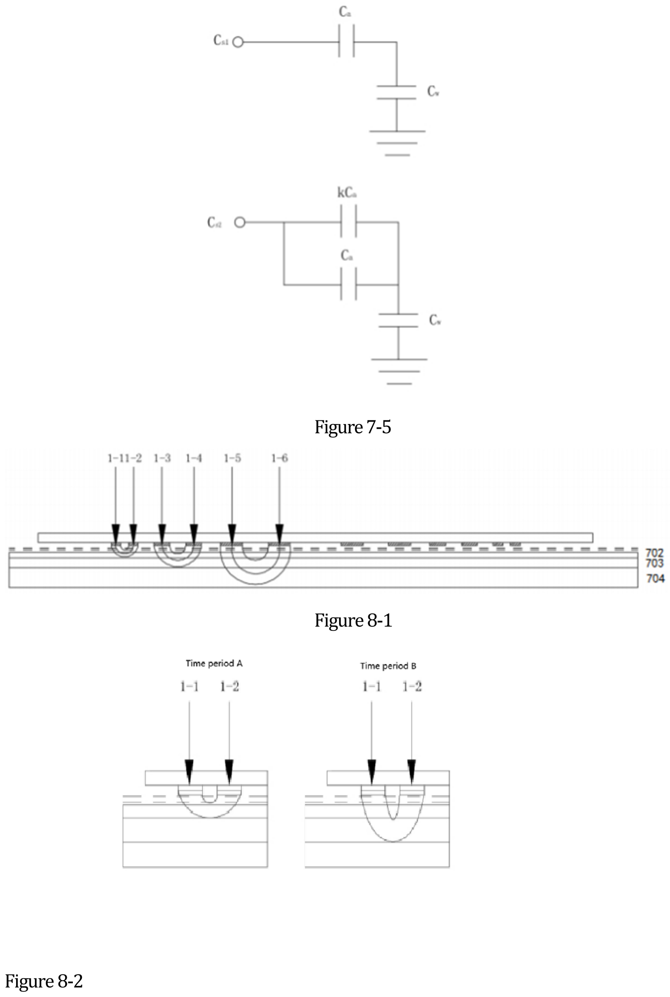

Figure 7-5 shows the combination of self-capacitance and variable area self-capacitance to measure $C_a$ The method, for example , 7-5 In the first measuring electrode$C_1$ The obtained self-capacitance measurement value Cs1 $_{is}$used as the first capacitance, and the equation Cs is obtained $_1 = \dfrac{C_a*C_w}{C_a+C_w}$ , Find $C_w = \dfrac{C_{s1}*C_a}{C_a-C_{s1}}$ , The second capacitance is configured as a third self-capacitance measurement value $C_{s2}$ , that is, the first measurement electrode $C_{s1}$ With the second measuring electrode$C_2$ Then through the second measuring electrode $C_{s2}$ Obtaining the self - capacitance, we can get the equation $\dfrac{1}{C_{s2}} = \dfrac{1}{C_a+k*C_a} + \dfrac{1}{C_w}$ , Find $CS_2 = \dfrac{(C_a+k*C_a)*C_w}{C_a+k*C_a+C_w}$ ,

Then the $C_a$ The calculation method is further configured as $C_a = \dfrac{C_{s1}*C_{s2}*k}{k*C_{s2}-k*C_{s1}+C_{s2}-C_{s1}}$ 。

The above $C_a$ These are all theoretical calculations. In practice, taking into account the test error and allowable tolerance, the $C_a$ The calculation ofk $_1$ k , $_2$ , where0.9-1.1, k $_2$ It is the allowable error value, such as $\pm$5% of the measured value.

As shownAs shown in 8-1, three electrode groups are configured, the first electrode group is electrode 1-1and 1-2 , the second electrode group consists of electrodes 1-3and 1-4The third electrode group consists of electrodes 1-5and 1-6 The three groups of electrodes have different areas and different depths of electric field lines, which can test skin components at different depths. The first electrode group has a small area and a shallow electric field line depth, which can test epidermal tissue. 702 The second electrode group has a moderate area and the depth of the electric field line is suitable for testing the dermis tissue. 703 skin components, the third electrode group has a large area and tests subcutaneous tissue 704 Similarly, the depth of the electric field lines can be changed by changing the spacing.

As shown8-2As shown, since different components of the skin react differently to excitation signals of different frequencies, at least two frequencies of excitation signals can be configured to detect different components within the same depth range. At the same time, the frequency difference of the excitation signal becomes larger, which can affect the distribution of the electric field lines and thus control the detection depth . A When the excitation frequency is the first excitation frequency, the depth of the electric field line can be measured to the epidermal tissue 702 The skin component capacitance value, in the period B When the excitation frequency is the second excitation frequency, the depth of the electric field line can be measured in the dermis tissue 703 skin component capacitance value, through the combination of different electrodes and different frequencies, the test range can be expanded and the test depth distinction can be more refined

As shown 2. Figure As shown in 9-1, the detection end 100 The skin component detection module includes a standard liquid reservoir170, standard liquid storage device 170 Mainly includes standard solution detection electrode 171. Standard liquid 172. Sealed cavity

173. The standard liquid detection electrode 171 can be a pair of detection electrodes, attached to opposite sides of the outer side of the sealed cavity. In the initial state, the measured initial capacitance value and the corresponding environmental variable factors will be recorded. When the environment changes , the capacitance value changes. The capacitance value change of the standard liquid detection electrode 171 can be input into the processing module to perform environmental correction on the test results. Can be water or standard grease , stored in a sealed cavity

173, the user detects the skin composition through the digital circuit CDC Comparing the capacitance value measured by the standard liquid detection electrode 171 with the capacitance value measured by the user can reduce the measurement error caused by changes in environmental factors. 172 Stored in sealed chamber 173 The internal part does not need to be frequently replaced, which can reduce the user's calibration operation during skin testing and simplify the operation.

As shown 10As shown, the detection end of the skin detection operation robot system 100, including at least 3 The processing module can be used to test the mutual capacitance value of different electrode combinations and test the skin components at different depths .702When the switchK1, K4, K20, K30 Closed or K5, K8, K20, K30 closure or When K9, K12, K20, and K30 are closed, the depth of the electric field line can measure the capacitance of the skin components at three different locations of the epidermis, and the average value can be obtained to reduce the measurement error caused by positioning error.703 When the switch K1, K3, K6, K8, K20, K30 Closed orK5, K7, K10, K12, K20, K30 Closed orK2, K4, K5, K7, K20, K30 Closed, the depth of the electric field line can measure the capacitance of the dermal tissue components at two different locations, and the average value can be obtained to reduce the measurement.

error caused by positioning error. 704When the switchK1, K3, K10, K12, K20, K30 Closed orK2, K4, K9, K11, K20, K30, the depth of the electric field line can measure the capacitance of the subcutaneous tissue skin component. By increasing the number of electrodes, multiple groups of subcutaneous tissue skin component capacitance values can be obtained, and then the average value can be obtained to reduce the measurement error caused by the positioning error .

Finally, it should be noted that the above embodiments are only used to illustrate the technical solutions of the present invention, rather than to limit the scope of protection of the present invention . Although the present invention has been described in detail with reference to the preferred embodiments, those skilled in the art should understand that the technical solutions of the present invention may be modified or replaced by equivalents without departing from the essence and scope of the technical solutions of the present invention.

## Claims

1. A skin detection and manipulation robot system with external pressure detection function, **characterized by**:

   it has a processing module, a detection end (100, an external actuator (200, an external pressure detection device (180, and an auxiliary positioning device for locating a specific part of the human body (300) ;
   the detection end (100) is provided with a probe for detecting the skin at a specific part of the human body, and a front end surface of the probe directly or indirectly abuts against the skin when the sensor for detecting the skin contacts the skin;
   the external pressure detection device (180) is configured to sense a first contact pressure between the front end face and the skin through the external pressure detection device (180) when the front end face directly or indirectly abuts against the skin ;
   the external actuator (200) is used to drive the front end surface to move so as to adjust the first contact pressure;
   the probe is provided with an internal pressure control device for controlling a second contact pressure between the sensor and the skin;
   the processing module is coupled to the external actuator (200) and the external pressure detection device (180) respectively.

2. According to the claim 1, the skin detection and operation robot system is **characterized by**:
   the probe is provided with an internal pressure detection device (140) coupled to the processing module, and the internal pressure detection device ( 140) is used to detect a second contact pressure between the sensor and the skin.

3. According to the claim 2, the skin detection and operation robot system is **characterized by**:
   the internal pressure detection device (140) is configured as a capacitive pressure sensing component.

4. According to the claim 1or2,the skin detection and operation robot system is **characterized by**:

the internal pressure control device is configured as an elastic body, and the sensor is fixed to the probe via the elastic body so as to elastically control the second contact pressure within a set range; or the internal pressure control device is configured as an internal actuator ( 110) coupled to the processor, and the internal actuator (110) is used to drive the sensor on the probe to move so as to adjust the second contact pressure between the sensor and the skin.

5. According to the claim 1, the skin detection and operation robot system is **characterized by**:

the auxiliary positioning device (300) is configured as a fixing bracket for assisting in fixing a specific part of the human body ; and/or the auxiliary positioning device (300) is configured as a visual scanning imaging system coupled to the processing module, the visual the visual scanning imaging system confirms the target detection position by scanning a specific part of the human body, and the processing module controls the movement of the external actuator (200) according to the target detection position.

6. According to claim 1, the skin detection and operation robot system is **characterized in that** the external actuator (200) is configured as a movable robotic arm (201.

7. According to the claim 1, the skin detection and operation robot system is **characterized by**: the number of the external pressure detection devices (180) is configured to be at least two, and the devices are arranged around the probe.

8. According to the claim 1, the skin detection and operation robot system is **characterized by**: the detection end (100) is provided with a standard connector, and the standard connector is used to be replaceably connected to different probes for detecting human skin so as to realize the corresponding detection function of the probe.

9. According to the claim 1, the skin detection and operation robot system is **characterized by**:

the skin detection and operation robot system is provided with a capacitance-to-digital conversion circuit; at least one of the probes is configured as a skin component detection module for detecting skin components, and a sensor of the skin component detection module is configured to include at least a first measuring electrode and a second measuring electrode; the first measuring electrode and the second measuring electrode are used to contact the skin through the insulating layer (122) , and the series capacitance $C_{a1}$ between the first measuring electrode and the human body and the series capacitance $C_{a2}$ between the second measuring electrode and the human body The ratio is configured to set a known proportionality factork; the capacitance-to-digital conversion circuit is coupled to each measuring electrode and obtains a first capacitance and a second capacitance , the first capacitance being configured as one of a first self-capacitance measurement value obtained by the first measuring electrode, a second self-capacitance measurement value obtained by the second measuring electrode, a third self-capacitance measurement value obtained by connecting the first measuring electrode and the second measuring electrode in parallel, and a first mutual capacitance measurement value obtained by the first measuring electrode and the second measuring electrode, and the second capacitance being configured as one of the remaining three; the processing module is used to construct a distributed capacitance of the human body to the ground based on the first capacitance$C_w$, the corresponding series capacitance as variables, and constructing the first equation based on the second capacitance with the human body to ground distribution $C_w$, the second equation with the corresponding series capacitance as a variable, using the equation group consisting of the first equation and the second equation and the proportional coefficient k Calculate the series capacitance $C_{a1}$ or series capacitors $C_{a2}$ To output the component information in the skin.

10. According to the claim9, the skin detection and operation robot system is **characterized in that** the distance between the first measuring electrode and the second measuring electrode is configured as0.1mm-2mm.

11. According to the claim9, the skin detection and operation robot system is **characterized in that**: the skin component detection module is provided with at least three measurement electrodes distributed at different positions; the capacitance-to-digital conversion circuit couples the measurement electrodes via an analog switch array to selectively combine any at least two measurement electrodes to form a pair of electrode groups for detecting mutual capacitance.

12. According to the claim 11, the skin component detection module is **characterized by**:
there are at least two pairs of electrode groups, wherein the electric field lines of the mutual capacitance formed by each pair of electrode groups have different depths.

13. According to claim 12, the skin component detection module is **characterized in that** the electrode group is configured to include at least two of the following:

a first electrode group, wherein the spacing and/or area of two electrodes in the first electrode group are configured so that the electric field lines of the mutual capacitance thereof can penetrate deep into the epidermal tissue (702) of the skin;
a second electrode group, wherein the spacing and/or area of two electrodes in the second electrode group are configured to enable the electric field lines of the mutual capacitance to penetrate deep into the dermis (703) of the skin;
the third electrode group, wherein the distance and/or area between two electrodes in the third electrode group are configured so that the electric field lines of the mutual capacitance can penetrate deep into the subcutaneous tissue (704) of the skin.

14. According to the claim 11, 12or 13, the skin detection and operation robot system is **characterized by**:
the excitation signal outputted by the capacitance-to-digital conversion circuit to the electrode group is configured to include at least two different frequencies.

15. According to claim 14, the skin detection and operation robot system is **characterized in that** each of the frequencies has different sensitivities to different components in the skin.

16. According to the claim9, the skin component detection module is **characterized by**:

the skin component detection module also includes a standard liquid storage device (170) ;
the standard liquid storage device (170) comprises at least:

a sealed cavity (173), a standard liquid (172) corresponding to the component for calibration or differential measurement, disposed within the sealed cavity (173), and a liquid detection electrode for measuring the capacitance value of the standard liquid (172) under different environmental conditions;
the capacitance-to-digital conversion circuit is coupled to the liquid detection electrode;
the processing module is configured to calibrate the series capacitor $C_{a1}$ or series capacitor $C_{a2}$ based on the capacitance value of the standard liquid (172).

Figure 1

Figure 2

Figure 3-1

Figure 3-2

Figure 4

304

302

301

303

Figure 5

160

160

Figure 6

$C_{s1}$   $C_{s2}$

121

122

Figure 7-1

$C_a$   $kC_a$

Human Body

$C_w$

Figure 7-2

Figure 7-3

Figure 7-4

$C_{s1}$ ○—||—$C_a$

$C_v$

$C_{s2}$ ○—||—$kC_a$

$C_a$

$C_v$

Figure 7-5

1-11-2  1-3  1-4  1-5  1-6

702
703
704

Figure 8-1

Time period A

1-1   1-2

Time period B

1-1   1-2

Figure 8-2

Figure 9-1

Figure 10

## INTERNATIONAL SEARCH REPORT

| | |
|---|---|
| | International application No. |
| | **PCT/CN2024/087865** |

| A. | CLASSIFICATION OF SUBJECT MATTER |
|---|---|

A61B5/00(2006.01)i; A61B5/0531(2021.01)i; A61B5/0537(2021.01)i; A61B34/32(2016.01)i; A61B90/00(2016.01)i; A61B34/10(2016.01)i; A61B34/20(2016.01)i; A61G15/00(2006.01)i; A61G15/10(2006.01)i; A61G15/12(2006.01)i

According to International Patent Classification (IPC) or to both national classification and IPC

| B. | FIELDS SEARCHED |
|---|---|

Minimum documentation searched (classification system followed by classification symbols)

IPC:A61B,A61G

Documentation searched other than minimum documentation to the extent that such documents are included in the fields searched

Electronic data base consulted during the international search (name of data base and, where practicable, search terms used)

CNTXT, CNABS, CNKI, WPABSC, ENTXT, DWPI: 北京他山科技, 孙滕谌, 曾凡佑, 王凯, 机器人, 机械臂, 机器臂, 皮肤, 组分, 弹性, 光泽, 油脂, 水分, 水份, 水量, 参数, 组成, 电阻, 阻抗, 电极, 检测, 监测, 测量, 压力, Robot+, Mechanical, Arm?, Skin, Parameter?, Elastic+, Moisture, Resistance, Impedance, Electrode?, Detect+, Measur+, Pressure

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 116548919 A (BEIJING TASHAN TECHNOLOGY CO., LTD.) 08 August 2023 (2023-08-08)<br>    description, paragraphs [0053]-[0068], and figures 1-10 | 1-16 |
| PX | CN 220089458 U (BEIJING TASHAN TECHNOLOGY CO., LTD.) 28 November 2023 (2023-11-28)<br>    description, paragraphs [0053]-[0068], and figures 1-10 | 1-16 |
| A | CN 109171647 A (SHANGHAI CHANGREN INFORMATION TECHNOLOGY CO., LTD.) 11 January 2019 (2019-01-11)<br>    description, paragraphs [0021]-[0025], and figure 1 | 1-16 |
| A | CN 214157285 U (CHANGSHA YAHAN MEDICAL COSMETIC HOSPITAL CO., LTD.) 10 September 2021 (2021-09-10)<br>    description, paragraphs [0022]-[0032], and figures 1-4 | 1-16 |
| A | CN 102066928 A (DREXEL UNIVERSITY et al.) 18 May 2011 (2011-05-18)<br>    entire document | 1-16 |

| | | | |
|---|---|---|---|
| ✓ Further documents are listed in the continuation of Box C. | | ✓ See patent family annex. | |

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **15 July 2024** | **19 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2024/087865** |

## C.    DOCUMENTS CONSIDERED TO BE RELEVANT

| Category* | Citation of document, with indication, where appropriate, of the relevant passages | Relevant to claim No. |
|---|---|---|
| A | CN 109124587 A (SHANGHAI CHANGREN INFORMATION TECHNOLOGY CO., LTD.) 04 January 2019 (2019-01-04) entire document | 1-16 |
| A | CN 109222897 A (CHINA-JAPAN FRIENDSHIP HOSPITAL) 18 January 2019 (2019-01-18) entire document | 1-16 |
| A | CN 115300795 A (GUANGZHOU QINGHUACI HEALTH TECHNOLOGY CO., LTD.) 08 November 2022 (2022-11-08) entire document | 1-16 |
| A | CN 2708308 Y (GAO HANQIN) 06 July 2005 (2005-07-06) entire document | 1-16 |
| A | DE 2912349 A1 (GEBR. LIEBISCH GMBH & CO. KG) 16 October 1980 (1980-10-16) entire document | 1-16 |
| A | JP 2009153727 A (KAO CORP.) 16 July 2009 (2009-07-16) entire document | 1-16 |

## INTERNATIONAL SEARCH REPORT
### Information on patent family members

International application No.

**PCT/CN2024/087865**

| Patent document cited in search report | | | Publication date (day/month/year) | Patent family member(s) | | | Publication date (day/month/year) |
|---|---|---|---|---|---|---|---|
| CN | 116548919 | A | 08 August 2023 | CN | 220089458 | U | 28 November 2023 |
| CN | 220089458 | U | 28 November 2023 | CN | 116548919 | A | 08 August 2023 |
| CN | 109171647 | A | 11 January 2019 | CN | 109171647 | B | 11 June 2021 |
| CN | 214157285 | U | 10 September 2021 | None | | | |
| CN | 102066928 | A | 18 May 2011 | US | 2015025418 | A1 | 22 January 2015 |
| | | | | US | 10076247 | B2 | 18 September 2018 |
| | | | | EP | 2281192 | A2 | 09 February 2011 |
| | | | | KR | 20110049748 | A | 12 May 2011 |
| | | | | WO | 2009140660 | A2 | 19 November 2009 |
| | | | | AU | 2009246115 | A1 | 19 November 2009 |
| | | | | US | 2014058260 | A1 | 27 February 2014 |
| | | | | US | 8845555 | B2 | 30 September 2014 |
| | | | | HK | 1216040 | A1 | 07 October 2016 |
| | | | | US | 2011172565 | A1 | 14 July 2011 |
| | | | | US | 8562546 | B2 | 22 October 2013 |
| | | | | CN | 102066928 | B | 05 August 2015 |
| CN | 109124587 | A | 04 January 2019 | None | | | |
| CN | 109222897 | A | 18 January 2019 | CN | 209122202 | U | 19 July 2019 |
| CN | 115300795 | A | 08 November 2022 | CN | 115300795 | B | 18 April 2023 |
| CN | 2708308 | Y | 06 July 2005 | None | | | |
| DE | 2912349 | A1 | 16 October 1980 | None | | | |
| JP | 2009153727 | A | 16 July 2009 | JP | 5107696 | B2 | 26 December 2012 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 88108905 A
- US 20020029924 A1
- CN 202223551426
- CN 201910370967
- US 4532937 A
- US 5119828 A
- DE 29700324 U1
- US 5134401 A
- CN 202110957486
- CN 202110956246

**Non-patent literature cited in the description**

- *Korean J Oral Med*, 2009, vol. 34 (3)